# EUROPEAN PATENT APPLICATION

(11) **EP 2 952 759 A1**
(43) Date of publication of application: **09.12.2015**
(21) Application number: 15166707.8
(22) Date of filing: 07.05.2015
(51) Int. Cl.: F16C 19/52, F16C 41/00, G01N 27/22, G01M 13/04, G01N 33/28, G01N 27/04, F16C 19/06, F16C 33/66

(54) **ROLLING BEARING AND SENSOR ASSEMBLY INCLUDING THE SAME**

(30) Priority: 05.06.2014 GB 201410010
(71) Applicant: Aktiebolaget SKF, 415 50 Göteborg (SE)
(72) Inventor: Murray, Brian, Aberdeen, Aberdeenshire AB31 4JR (GB); Pallister, Dave M., Highland, MI Michigan 48357 (US)
(74) Representative: Tweedlie, Diane Harkness

(57) **Abstract**

The invention relates to a rolling bearing including at least two bearing rings (10, 12) and one set of rolling elements arranged in a space between the bearing rings (10, 12), wherein said space between the bearing rings (10, 12) is filled with grease (16), wherein at least one of the bearing rings (10, 12) and/or the rolling elements (14) is made of steel.

It is proposed to provide the bearing with at least one electrode (18) made from a non-ferrous material, whereby at least one surface of said electrode (18) is in contact with the grease (16), and at least one of the bearing rings (12) includes means for connecting a sensor wire (20) for applying a voltage between the bearing ring (12) and the electrode (18).

## Description

### Technical Field

The invention relates to grease lubricated rolling bearings, to a sensor assembly and to a method for detecting corrosion in grease lubricated bearings.

### Technical Background

Corrosion is a significant cause of failure of greased bearings in many industrial applications. Today, there is no practical way of detecting the risk of corrosion in a bearing in service.

The standard procedure is to extract a grease sample and to examine the grease in a laboratory. However, there is no guarantee that the sample will be representative of the grease pack, and the measured moisture content will be affected by the passage of time and ambient temperature changes from site to laboratory. Grease sampling is widely undertaken, but remains a poor preventative measure for corrosion.

Recently, there have been attempts to introduce a laser device that inspects the grease optically, and claims to detect the water content. There are doubts about the efficacy of this fairly complex and expensive technology.

### Summary of the Invention

The invention seeks to provide a rolling bearing and a sensor assembly including a rolling bearing enabling a reliable corrosion detection system for grease lubricated rolling bearings that is preferably also sensitive to detecting water contamination of the grease lubricant.

The invention relates to a rolling bearing including at least two bearing rings ant one set of rolling elements arranged in a space between the bearing rings, wherein said space between the bearing rings is filled with grease, wherein at least one of the bearing rings is made of steel.

In order to enable a reliable sensing of the risk and/or speed of corrosion, it is proposed that the rolling bearing comprises at least one electrode made from a non-ferrous material, wherein at least one surface of said electrode is in contact with the grease. The non-ferrous material is preferably used as a cathode and could be copper, carbon, or zinc, and in the form of a plate or ring extending around the bearing. In a simple and cost-saving embodiment, the electrode could be a copper wire extending around the bearing. In any case, the electrode has to be insulated from at least one of the bearing rings serving as the second electrode, preferably, the anode, of the sensor assembly employing the non-ferrous electrode.

The bearing ring and steel shafts are generally constructed with materials of lower electrochemical nobility and serve as a sacrificial source of electron for the corrosion reactions. Monitoring the current flow between the electrode and the bearing ring gives an indication of the rate of corrosion occurring in a bearing.

In typical oxidation reactions taking place in grease lubricated bearings, iron and steel spontaneously oxidize on an anode to Fe+2 if a cathodic reaction is present in the electrochemical cell formed by the electrode and one of the bearing rings. This results in an electromotive force. However, current will not flow in this electrochemical cell until a cathodic reaction occurs. Several reactions can occur at the cathode allowing current to flow. Water or hydronium ion (H₃O₊) can be reduced to form hydrogen gas. Oxygen can also be reduced in the presence of water (forming OH₋) or Fe₊₂ or Fe₊₃ can be reduced. All these reactions require the presence of water or oxygen in the grease such that essentially no current flows until the bearing is wet or corroding. Corrosion produces an abundance of electrochemically active species that increases current flowing in the cell. The non-ferrous electrode can absorb the electrons from the reduction reaction and lead to current densities enabling a reliable detection even in the presence of grease between the electrodes.

A sensor and monitoring system may be realized that detects the occurrence of corrosion within a greased bearing as defined above by detecting changes in the voltage/current characteristics between a non-ferrous electrode and the stationary ring of a the bearing. As the moisture content of a grease increases, the electrical resistance falls. This can give an indication of the risk of corrosion, but the measurement can be confused by other factors affecting the electrical resistance.

It is further proposed that said one surface of the electrode faces the rolling elements with a gap filled with grease there between. It is important to maintain thin films of grease between the anode and cathode of this system. The film of grease should be representative of the active lubricant in the rolling bearing. As a consequence, the grease between the cathode and the rolling bearing serving as the anode needs to be renewed or resampled continuously and the film thickness needs to be kept at a minimum. The positioning of the electrode close to the rolling elements is very advantageous for achieving this goal because the passing rolling elements will continuously move and replace the grease in the gap.

According to the invention, at least one of the bearing rings includes means for connecting a sensor wire for applying a voltage between the bearing ring and the electrode. The means could be a wire, a terminal for a wire or a connector for connecting the wire.

In a simple and cost-saving embodiment of the invention, the electrode is made of copper. Zinc or carbon electrodes may be suitable alternatives depending on the specific application.

According to a further aspect of the invention, the rolling bearing comprises a seal, wherein said electrode is integrated in the seal. This integration leads to a compact and robust design. It is further proposed that the electrode is formed as a conductive elastomer ring, which may be made of a carbon-loaded elastomer material.

A further aspect of the invention relates to a sensor assembly including a rolling bearing according to one of the preceding claims and detector circuit connected to the electrode and to at least one of the bearing rings, wherein the detector circuit is configured to measure currents flowing through the grease between the electrode and the bearing ring. The electrode or the electrodes are electrically connected via a current sensor and optionally a variable voltage source to the bearing ring. The detector circuit is formed as or acts similar to an amperometric detector, in particular, the detector circuit may be a picoammeter, i.e. an amperemeter suitable for measuring currents of the order of magnitude of 10⁻¹² A.

According to a further aspect of the invention, the sensor assembly is configured to apply a negative bias voltage to the electrode. As explained in further detail below, the inventors have found that a negative bias voltage may lead to an increase of corrosion currents under wet conditions by orders of magnitude. Preferably, the negative bias voltage has a value between -0,5 and -1,5V, preferably roughly 1 V. The bias current is measures with reference to the potential of the bearing rings, which are generally but not necessarily grounded.

The application of potential to the working electrode increases the sensitivity of the corrosion detection system. Simply connecting a picoammeter to the copper cathode/wet grease/bearing steel anode does not provide enough signal difference between dry systems and the wet corroding condition of the test bearings. This is due to the current/voltage response of this system. A picoammeter will be used without bias voltage measure current very close to the electrochemical equivalent to an isosbestic point for wet, dry and corroding bearings. Using an amperometric detector and an applied voltage of approximately -1 Volt to the cathode provides over 20-fold increases in corrosion currents for wet, corroding systems and enables a differentiatiation between wet and dry and corroding rolling bearings based on the magnitude of the measured current.

In a preferred embodiment of the invention, the sensor assembly includes a microprocessor configured to determine a parameter indicating a corrosion risk of the bearing from the signals from the amperometric detector circuit.

Further, it is proposed that the sensor assembly includes means for evaluating the parameter indicating a corrosion risk and means outputting a warning signal if the evaluation indicates a high corrosion risk. The means for outputting the warning signal could be formed as signal transmitting means or as a visual or optical warning means such as a light emitting diode.

In a preferred embodiment of the invention, the sensor assembly includes an electronic circuit including at least one of the amperometric detector circuit or the microprocessor, wherein said electronic circuit is fitted on a seal of the bearing. The integration enables a robust and compact design.

It is further proposed that the electronic circuit includes means for generating electrical energy form movements of the bearing and a wireless transmitter for transmitting signals from the amperometric detector circuit or the microprocessor. The means for generating electrical energy may be a harvester using the vibrations or oscillations of the passing rolling elements and may include piezo elements or coils suitable for this purpose.

The electrochemical cell used as a corrosion detection system uses the rolling bearing as an anode and a copper sheet as the cathode with grease as the medium between the two electrodes. The electronics for sensing water contamination and corrosion of rolling bearings is simply an amperometric detector known from the field of chromatography. This circuit allows the application of a potential to the working electrode (copper electrode) while maintaining the bearing as an auxiliary electrode (ground). By detecting corrosion early, preventative measures can be put in place to avoid bearing failure.

A further aspect of the invention relates to a method for detecting corrosion in grease lubricated bearings of the type described above. It is proposed that the electrode is used to measure an electrical current carried by electro-active species in the grease and to determine at least one parameter relating to corrosion processes of the bearing based on the measured current. In a preferred embodiment, the method includes applying a negative bias voltage as described above to the electrode.

The above description of the invention as well as the appended claims, figures and the following description of preferred embodiments show multiple characterizing features of the invention in specific combinations. The skilled person will easily be able to consider further combinations or sub-combinations of these features in order to adapt the invention as defined in the claims to his or her specific needs.

### Brief Description of the Drawings

- Fig. 1: is a schematic representation of a sensor assembly including a bearing according to the invention;
- Fig. 2: is a semi-logarithmic graph showing a Tafel plot analysis of the system of Fig. 1; and
- Fig. 3: is a linear plot equivalent to the Tafel plot of Fig. 2.

### Detailed Description of the Embodiments

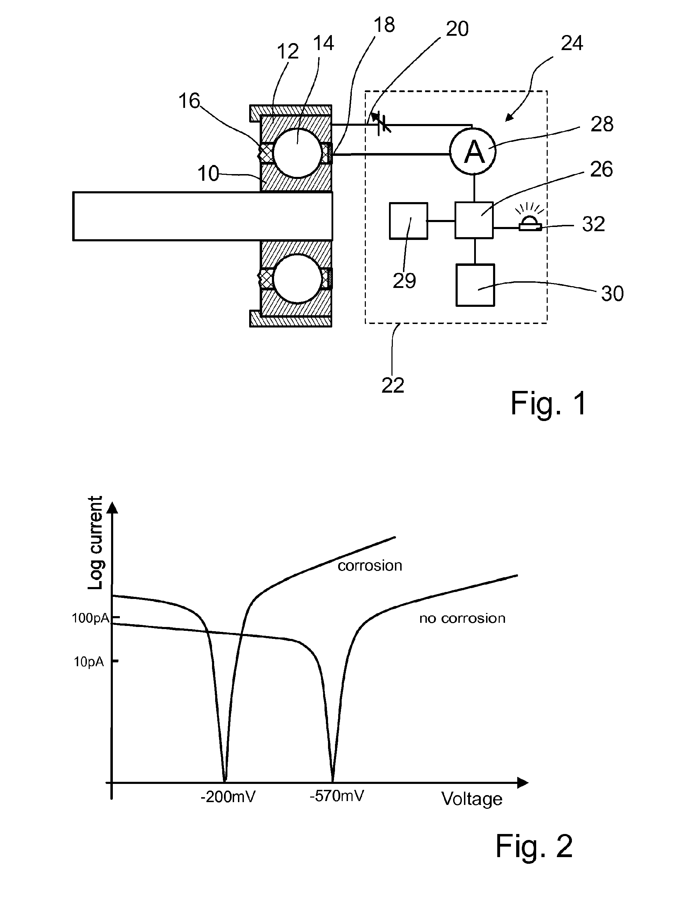

Fig. 1 illustrates a rolling bearing according to the invention. The bearing includes two bearing rings, i.e. an outer ring 10 and an inner ring 12, and one set of rolling elements 14 arranged in a space between the bearing rings 10, 12. The bearing is a grease lubricated bearing and the space between the bearing rings 10, 12 accommodating the rolling elements 14 is filled with grease 16 besides of the rolling elements 14. The bearing rings 10, 12 and the rolling elements 14 are made of standard electrically conducting bearing steel.

The rolling bearing comprises at least one electrode 18 made from copper and a surface of the electrode facing the space between the rings is in direct contact and completely covered with the grease 16 and the grease 16 fills the space between the inner ring 10, the outer ring 12 and the rolling elements 14 as well as an axial gap between the rolling elements 14 and the working electrode 18 completely. The copper working electrode 18 has the form of a plate or ring extending around the bearing. The electrode 18 is insulated from the grounded bearing rings 10, 12 and from the rolling elements 14 formed as balls. The outer ring 12 of the bearing serves as the second electrode, the anode.

As a consequence of the high conductivity of the rolling elements 14 and of the bearing rings 10, 12, the ohmic resistance between the copper electrode 18 and the outer ring 12 serving as the anode is essentially determined by the conductivity or resistivity of the grease layer between the electrode 18 and the rolling elements 14 and the grease film between the rolling elements 14 and the outer ring 12. These portions of the grease pack 16 of the bearing are continuously exchanged due to the movement of the rolling elements 14 such that the grease 16 in these portions is a reliable and representative sample of the active grease of the bearing.

Though this is not explicitly illustrated, the bearing includes a seal avoiding a loss of the lubricant and the electrode 18 is integrated in the seal or fixed thereto. The backside of the electrode 18 facing away from the rolling elements 14 is not exposed to the grease 16.

A sensor wire 20 for applying a voltage between the bearing ring 12 and the electrode 18 is connected to the outer ring 12 of the bearing by welding or by other suitable means.

A sensor assembly 22 formed as an electronic circuit including a detector circuit 24 and a microprocessor 26 is connected to the rolling bearing via the sensor wire 20 and includes an amperometric detector 28 connected to the electrode 18 and to at the outer ring 12. The amperometric detector 28 is configured to measure currents flowing through the grease 16 between the electrode 18 and the bearing ring 12. The sensor 22 assembly is configured to apply a negative bias voltage of -1 V to the electrode 18. The electronic circuit 22 is fitted on the seal of the bearing (not illustrated). The sensor assembly 22 employs the rolling bearing as the anode and the copper sheet 18 as the cathode with grease 16 as the medium between the two electrodes as the electrochemical cell of the corrosion detection system.

The measurement process is executed by the microprocessor 26 reporting the corrosion risk via wireless communication unit 30. The electronic circuit further includes power harvesting means 29 provided in the bearing to generate the energy required by the microprocessor 26 or for the wireless communication unit 30. The microprocessor 26 calculates and evaluates a parameter indicating a corrosion risk and outputs a warning signal via suitable means 32 such as an LED, if the evaluation indicates a high corrosion risk or corrosion hazard.

The invention is based on the principle that if a voltage equal and opposite to the electrochemical electrode potential difference that exits between the non-ferrous electrode 18 and the (ferrous) bearing ring 12, the current flowing is reduced to zero. This voltage changes when corrosion occurs on the bearing ring because of the chemical reaction that takes place. Detecting and monitoring the null voltage, i.e. the voltage where no current flow, therefore provides an unambiguous indication of the occurrence of corrosion. This voltage is equal and opposite of the electrode potential existing between the electrode material and the bearing steel. The null point is independent of the applied voltage. The null point is a good reference point for the operation of the detector.

A Tafel analysis of this system was performed to provide more information on the rolling bearing corrosion detection system. The plot in Fig. 2 is often referred to as a Tafel plot and is shown on a logarithmic scale, and used to characterise the behaviour of an electrodes and electrochemical species in an electrochemical cell. The plot shows the absolute value of the current versus the voltage such that the zero crossing appears as a negative singularity in the logarithmic plot.

The currents involved are extremely low- of the order 10₋₉ amps. As the applied voltage is swept through range from approximately -1 V to +1 V, the detected current will change, as indicated in the diagram in Fig. 2. There is a charging current associated with the polarization of the electrodes. Once the electrodes are polarized (charged), no current will flow through the amperometric cell until an electro-active species is present. The zero voltage will gradually shift towards higher, i.e. less negative values with increasing concentrations of electro-active species.

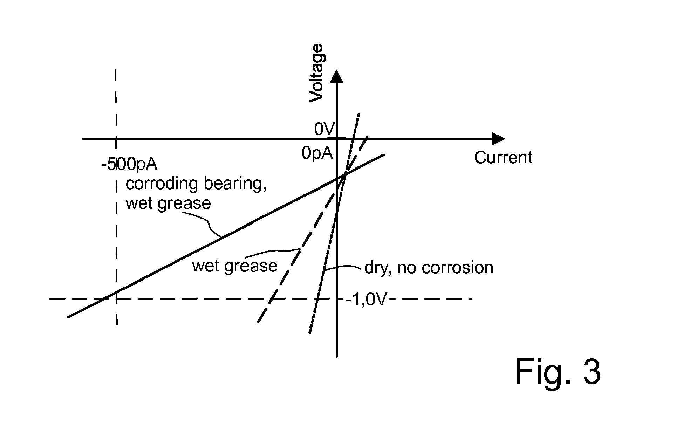

Fig. 3 represents the linear plot equivalent to Fig. 2. Besides of the null voltage, the sensor circuit measures the slope of the voltage/current graph in Fig. 3 near the null voltage. This slope indicates the electrical resistance of the grease pack. Polarizing the cathode by the negative bias voltage of -1 V increases the sensitivity of the electrochemical cell towards water and rust contamination.

Changes in temperature, moisture or salt content of the grease will affect the slope IR. This resistance is also influenced by the type of grease and the geometry of the grease pack.

The most important observation of Figure 3 is the amplification of corrosion currents, when a potential is applied to cathode. Applying -1 volt results in signal currents greater than 400 pA. This is a twentyfold increase in signal as compared to the values observed without voltage bias (14 pA). Also there are large differences in the galvanic currents observed for dry non-corroding system (dotted trace) as compared to the wet non corroding system (dash trace) and the actively corroding system (solid trace).

The results of the Tafel Plot analysis of samples are shown in the following table:

| Sample | Ecorr | Icorr | Beta A | Beta C |
|---|---|---|---|---|
| Dry grease, dry bearing, no corrosion | -508 mV | 14.9 pA | 0.40 V/decade | 0.44 V/decade |
| Wet grease, dry bearing, no corrosion | -300 mV | 101.0 pA | 0.51 V/decade | -0.51 V/decade |
| Wet grease, wet bearing, no corrosion | -273 mV | 82.8 pA | 0.51 V/decade | -1.09 V/decade |
| Wet grease, wet bearing, active corrosion | -184 mV | 427.0 pA | 0.64 V/decade | -1.29 V/decade |

Ecorr represents the zero current voltage of the system, Beta A represents the asymptotic slope of the Tafel plot of the current on the negative voltage side and Beta C represents the asymptotic slope of the Tafel plot of the current on the positive voltage side Icorr is a quantiy representative of the magnitude of the current measured, which is defined as the current value of a crossing point between the asymptotic straight lines in the graphs.

Ecorr is the corrosion potential of the sample or cell, the potential of a corroding surface in an electrolyte. It defines the cell potential and whether potential lies in a region of corrosion activity or passivity.

This circuit allows the application of a potential to the working electrode 18 (copper electrode) while maintaining the bearing as an auxiliary electrode 12 (ground). The current follower in this circuit measures the corrosion current from the detector. The application of potential to the working copper electrode 18 increases the sensitivity of the corrosion detection system. Simply connecting a picoammeter to the copper cathode/wet grease/bearing steel anode would provide a much smaller signal difference between dry systems and the wet corroding condition of the test bearings.

As described above, the invention proposes to use an electrochemical cell used as a corrosion detection system, wherein the electrochemical cell uses the rolling bearing as an anode and a copper sheet as the cathode with grease 16 as the medium between the two electrodes 12, 18. An amperometric detector 24 is used to apply a potential to the copper cathode 18 of the rolling bearing detector in order to amplify the difference between rolling bearing corrosion, the conditions of wet grease or a wet bearing and dry conditions based on current measurements.

## Claims

1. Rolling bearing including at least two bearing rings (10,12) and one set of rolling elements (14) arranged in a space between the bearing rings (10, 12), wherein said space between the bearing rings is filled with grease (16), wherein at least one of the bearing rings (10, 12) is made of a ferrous material, the bearing further comprising at least one electrode (18) made from a non-ferrous material, wherein at least one surface of said electrode (18) is in contact with the grease (16),
**characterized in that** at least one of the bearing rings (12) includes means for connecting a sensor wire (20) for applying a voltage between the bearing ring (12) and the electrode (18).

2. Rolling bearing according to one of the preceding claims,
**characterized in that** said one surface of the electrode (18) faces the rolling elements (14) with a gap filled with grease (16) there between.

3. Rolling bearing according to one of the preceding claims,
**characterized in that** the electrode (18) is made of copper.

4. Rolling bearing according to one of the preceding claims,
**characterized by** comprising a seal, wherein said electrode (18) is integrated in the seal.

5. Rolling bearing according to claim 4,
**characterized in that** the electrode (18) is formed as a conductive elastomer ring.

6. Sensor assembly including a rolling bearing according to one of the preceding claims and an amperometric detector circuit (28) connected to the electrode (18) and to at least one of the bearing rings (12), wherein the amperometric detector circuit (28) is configured to measure currents flowing through the grease (16) between the electrode (18) and the bearing ring (12).

7. Sensor assembly according to claim 6,
**characterized in that** the sensor assembly is configured to apply a negative bias voltage to the electrode (18).

8. Sensor assembly according to claim 7,
**characterized in that** the negative bias voltage has a value between -0,5 and - 1,5V.

9. Sensor assembly according to one of claims 6 - 8,
**characterized by** comprising a microprocessor (26) configured to determine a parameter indicating a corrosion risk of the bearing from the signals from the amperometric detector circuit (24).

10. Sensor assembly according to claim 9,
**characterized by** comprising means (26) for evaluating the parameter indicating a corrosion risk and means outputting a warning signal if the evaluation indicates a high corrosion risk.

11. Sensor assembly according to one of claims 6 - 10,
**characterized by** comprising an electronic circuit (24) including at least one of the amperometric detector circuit (28) or the microprocessor (26), wherein said electronic circuit is fitted on a seal of the bearing.

12. Sensor assembly according to claim 11,
**characterized in that** the electronic circuit includes means (29) for generating electrical energy form movements of the bearing and a wireless transmitter (30) for transmitting signals from the amperometric detector circuit (28) or the microprocessor (26).

13. Method for detecting corrosion in a grease lubricated bearing according to one of claims 1 - 5, **characterized in that** the electrode (18) is used to measure an electrical current carried by electro-active species in the grease (16) and to determine at least one parameter relating to corrosion processes of the bearing based on the measured current.

14. Method according to claim 13, further including applying a negative bias voltage to the electrode (18).
